# EUROPEAN PATENT APPLICATION

(11) **EP 4 670 726 A1**
(43) Date of publication of application: **31.12.2025**
(21) Application number: 24386077.2
(22) Date of filing: 28.06.2024
(51) Int. Cl.: A61K 35/747, A61P 25/28, A61K 31/702, A61K 35/00, A23L 33/00

(54) **COMPOSITION FOR IMPROVING GUT HEALTH ASSOCIATED WITH A GUT-BRAIN-AXIS CONDITION**

(71) Applicant: N.V. Nutricia, 2712 HM Zoetermeer (NL)
(72) Inventor: Kostopoulos, Ioannis, 3584 CT Utrecht (NL); Kumar, Himanshu, 3584 CT Utrecht (NL); Roeselers, Guus, 3584 CT Utrecht (NL)
(74) Representative: Yazitzoglou, Evagelia S.

(57) **Abstract**

The present invention refers to a combination of prebiotic fibres with probiotic bacteria for use in preventing and treating gut brain axis-associated conditions, and compositions comprising the same. In particular, the combination comprises the prebiotic fibres GOS and FOS and probiotic strains of *Lacticaseibacillus paracasei.*

## Description

### FIELD OF THE INVENTION

The present invention refers to a combination of prebiotic fibres with probiotic bacteria for use in preventing and treating impaired gut health associated with a gut-brain-axis condition in a subject in need thereof, and compositions comprising the same. In particular, the combination comprises the prebiotic fibres GOS and FOS and probiotic species of *Lacticaseibacillus.*

### BACKGROUND OF THE INVENTION

There is a bi-directional communication pathway between the gut and the brain called the microbiota-gut-brain axis. Brain function is influenced by the gut microbiota via neuroendocrine, neuroimmune and autonomic nervous systems (Mayer, E. A. Gut feelings: the emerging biology of gut-brain communication. Nat. Rev. Neurosci. 12, 453-466, 2011). An imbalance in the gut microbiota results in or affects the co-ordination of the microbiota-gut-brain axis in human health.

Altered gut microbiota in ASD children has been reported to include a diminished presence of *Bacteroides, Lactobacillus,* and *Bifidobacterium* (Kim, J. "Autism Spectrum Disorder and Eating Problems: The Imbalance of Gut Microbiota and the Gut-Brain Axis Hypothesis". J Korean Acad Child Adolesc Psychiatry, 2024, 35(1):51-56). Moreover, it has been hypothesized that the degree of microbial alteration correlates with the severity of the disease since faecal microbiota and metabolomes alterations were higher in atypical children compared to neurotypical children (De Angelis et al. "Autism spectrum disorders and intestinal microbiota". Gut Microbes, 6:3, 207--213; May/June 2015), and that ASD children present a much higher ratio of Enterobacteriaceae to Bifidobacteriaceae (Coretti et al. "Gut Microbiota Features in Young Children with Autism Spectrum Disorders" Frontiers in Microbiology, 2018, volume 9, article 3146). *Veillonella* is found to be more abundant in neurotypical children than in ASD children (Ye et al. Comparison of gut microbiota in autism spectrum disorders and neurotypical boys in China: A case-control study. Synth Syst Biotechnol. 2021 May 21;6(2):120-126; Strati et al. New evidences on the altered gut microbiota in autism spectrum disorders. Microbiome 5, 24 (2017)).

Impaired protein digestion has been reported in patients suffering from AD. Sanctuary et al. ("Dietary Considerations in Autism Spectrum Disorders [ASD]: The Potential Role of Protein Digestion and Microbial Putrefaction in the Gut-Brain Axis", Front. Nutr., vol. 5, 2018) reports that in children with ASD, reduced proteolytic capacity may cause gastrointestinal problems and also exacerbate ASD symptoms.

Supplementation with probiotics has suggested an amelioration of ASD' symptoms (Sivamaruthi et al. The Role of Microbiome, Dietary Supplements, and Probiotics in Autism Spectrum Disorder Int. J. Environ. Res. Public Health 2020, 17(8), 2647). Other probiotic mixtures have also been suggested to improve depression and anxiety but not schizophrenia, thereby highlighting the specificity of the effects generated by probiotics in each microbiota state (Mörkl et al. Probiotics and the Microbiota-Gut-Brain Axis: Focus on Psychiatry. Curr Nutr Rep 9, 171-182 (2020)). *Bifidobacterium* has been proposed as a "psychobiotic" for its ability to produce neuromodulators and to influence gut-brain relationship through interaction with other commensal bacteria (Sarkar et al, 2016).

A synbiotic preparation comprising a mixture of probiotics strains in a total concentration of 1 × 10¹⁰ CFU/day (5.0 × 10⁹ CFU of *Lactobacillus paracasei* HII01 and 5.0 × 10⁹ CFU of *Bifidobacterium animalis* subsp. *lactis*) and 10 g prebiotics (5 g galacto-oligosaccharides (GOS), and 5 g oligofructose (FOS)) has been proposed to attenuate cortisol levels in stressed population (as determined by 'Thai Stress Test'), reduced negative feelings, and altered levels of IL-10, LPS and IgA (Lalitsuradej et al. "The Effects of Synbiotics Administration on Stress-Related Parameters in Thai Subjects-A Preliminary Study", Foods, 2022, 11, 759).

Wang et al 2019 ("Prebiotic supplementation of in vitro fecal fermentations inhibits proteolysis by gut bacteria, and host diet shapes gut bacterial metabolism and response to intervention. Appl Environ Microbiol 85:e02749-18) reports the impact of prebiotics on proteolysis within the gut. It was found that addition of fructan prebiotic Synergy1 in faecal slurries increased the levels of bifidobacteria, and lowered the production of ammonia and branched-chain fatty acids (BCFA).

There remains a need for improved compositions to prevent and treat gut brain axis-associated conditions by targeting key elements related to such conditions.

### SUMMARY OF THE INVENTION

To test the effect of the combination according to the invention in dysbiosis induced by proteolytic fermentation, faecal samples from healthy children were used in an *in vitro* fermentation model to induce proteolysis thereby mimicking the dysbiotic pattern found in the gut microbiota of neurodivergent individuals. It has been unexpectedly found that the combination of the invention is able to modulate key bacterial species for gut health in an impaired gut microbiota environment such as that found in subjects suffering from ASD.

Accordingly, a first aspect of the invention relates to a combination of galacto-oligosaccharides and fructo-oligosaccharides with *Lacticaseibacillus ssp.* for use in preventing and/or treating impaired gut health associated with a gut-brain-axis condition in a subject in need thereof.

Advantageously, the compositions of the invention are capable of substantially delay the increase in the ratio of relative abundance of Enterobacteria to Bifidobacteria as compared to a reference synbiotic composition in neurodivergent-like gut microbiota. The present invention thus provides a safe and efficient therapeutic tool for the management of gut brain axis-associated conditions, particularly in individuals with impaired protein digestion.

A second aspect of the invention relates to a composition comprising the combination of prebiotics and probiotics as herein defined.

### DETAILED DESCRIPTION OF THE INVENTION

The present invention concerns a combination of prebiotic fibre(s) selected from galacto-oligosaccharides and fructo-oligosaccharides with a probiotic strain(s) of *Lacticaseibacillus ssp.* for use in preventing and/or treating impaired gut health associated with a gut-brain-axis condition in a subject in need thereof.

In some jurisdictions the invention can also be worded as the use of prebiotic fibre(s) selected from galacto-oligosaccharides and fructo-oligosaccharides and of probiotic strain(s) of *Lacticaseibacillus ssp.* in the manufacture of a composition for use in preventing and/or treating impaired gut health associated with a gut-brain-axis condition in a subject in need thereof.

In further jurisdictions, the invention can also be worded as a method for use in preventing and/or treating impaired gut health associated with a gut-brain-axis condition in a subject in need thereof by administering a combination of prebiotic fibre(s) selected from galacto-oligosaccharides and fructo-oligosaccharides with probiotic strain(s) of *Lacticaseibacillus ssp.* to the subject.

"Impaired gut health associated with a gut-brain-axis condition" refers to altered gut microbiota or gut function as prevalently found in subjects diagnosed with, or subjects likely to develop, gut brain axis conditions. It has been reported that impaired gut function in these subjects lead not only to worsening of GI symptoms and but also linked to the onset and exacerbation of the neurodevelopmental condition (Sanctuary et al. 2018). In these subjects, microbial protein fermentation and inflammatory putrefactive metabolites are increased, whereas the levels of fiber-fermenting bacteria and bacterial diversity are reduced.

"Impaired gut health" can be routinely diagnosed by a medical professional. "Impaired gut health" is preferably selected from gut dysbiosis, gut dysfunction, fragile gut, and/or impaired protein digestion.

"Gut-brain-axis conditions" according to the invention can be clinically diagnosed as neurodevelopmental/neurodivergent disorders. Preferably, the gut brain axis conditions are selected from autism spectrum disorders (ASDs) or attention deficit hyperactivity disorder (ADHD). The ASDs are selected from autism disorder (AD), Asperger's syndrome and pervasive developmental disorder-not otherwise specified (PDD-NOS). In a preferred embodiment, the gut brain axis condition is autism disorder (AD).

The subject is preferably a neurodivergent patient or a subject that is more likely to develop a neurodevelopmental disorder, such as a subject having a first degree relative already diagnosed as neurodivergent. Accordingly, the subject is preferably selected from a neurodivergent subject or a subject which has a neurodivergent sibling, more preferably the subject is a neurodivergent subject.

The subject is an infant (0-12 months of age), toddler (12-36 months of age), child (3-12 years of age), teenager (12-18 years of age) or an adult (more than 18 years of age). Preferably, the subject is a toddler, child, teenager or an adult, more preferably, a child or teenager.

"Gut dysbiosis" refers to a medical condition characterized by the imbalance of the gut microbial profile, *i.e*., the increase of pathogenic bacteria and/or decrease of beneficial bacteria. Gut dysbiosis may be transitory or permanent. For instance, transitory gut dysbiosis may occur over the course and/or following antibiotic therapy, but gut microbiota balance may be re-established on its own or as a response to dedicated therapy. Permanent gut dysbiosis may be identified in patients co-suffering from e.g. ASDs, inflammatory bowel syndrome, etc. In a preferred embodiment, the subject further suffers from permanent gut dysbiosis.

"Gut dysfunction" associated with a gut-brain-axis condition includes impaired gut permeability, reduction in effectiveness of digestion, and impaired nutrients absorption and uptake.

"Fragile gut" is defined as a gastrointestinal tract that is basically "healthy" (i.e., lack of defined gastrointestinal disease), but is not robust to stressors, challenges, or microbial insults such as indigestible or toxic components or infectious agents.

"Impaired protein digestion" is defined as a shift towards a proteolytic gut ecosystem favouring thus proteolytic fermentation, and is commonly identified in individuals suffering from gut brain axis-associated conditions. Proteolytic fermentation is the process in which proteins and/or peptides are anaerobically broken down by microorganisms in the gastro-intestinal tract of mammals. An increased level of proteobacteria, *i.e.,* proteolytic bacteria, in the gastro-intestinal tract is considered an indicator of increased proteolytic fermentation, or the other way around a reduced level of proteobacteria, *i.e.,* proteolytic bacteria, in the gastro-intestinal tract is considered an indicator of reduced proteolytic fermentation. There is mounting evidence that an increase in proteobacteria is linked to the appearance and development of ASDs and, conversely, reducing the relative abundance thereof prevents and ameliorates the symptoms of gut brain axis-associated conditions, such as ASDs.

According to one embodiment, the subject is diagnosed to suffer from at least one atypical eating behaviour. Atypical eating behaviours are herein defined include one or more of clinically identified food refusal patterns, food dislike patterns, food selectivity, limited food preference/repertoire, obsessive eating pattern, pica, pocketed food, anorexia, etc. Atypical eating behaviour and disorders can be clinically diagnosed and are well documented, such as in the "Clinical Handbook of Complex and Atypical Eating Disorders" (edited by Leslie K. Anderson, Stuart B. Murray, Walter H. Kaye, Oxford University Press, 2018).

In one embodiment, preventing and/or treating impaired gut health associated with a gut-brain-axis condition comprises improving the gut microbiota of the subject. More preferably, improving gut microbiota comprises at least one of:
a. increasing the relative abundance of saccharolytic bacterial taxa, preferably selected from Bifidobacteriaceae, Lactobacillaceae, Lachnospiraceae and Veillonellaceae;
b. decreasing the relative abundance of proteolytic bacterial taxa, preferably selected from Enterobacterales, Peptostreptococcaceae, Peptostreptococcales-tissirierallales, Oscillospiraceae and Eggerthellaceae;
c. decreasing the ratio of Enterobacterales to Bifidobacteriaceae; or
d. increasing the ratio of Veillonellaceae plus Bifidobacteriaceae to Enterobacterales.

Relative abundance of gut bacteria in the gut microbiota, as herein referred, can be suitably determined in sample stools of a subject using microbiomics techniques known in the art, such as amplification kits commercially available and sequencing using 16S rRNA gene sequencing protocols. Such techniques are increasingly used in medical practice to confirm clinical diagnostic of gut dysbiosis, impaired gut microbiota, and associated conditions.

In the context of the present invention, increasing or reducing relative abundance is compared to a control which the reference value from a subject that has not been administered the combination according to the invention. Increase or decrease of relative abundance refers to a difference of at least 10% compared to control, more preferably at least 20% compared to control, more preferably at least 40% compared to control, more preferably at least 60% compared to control.

The combination according to the invention is also referred to as a synbiotic. As used herein, "synbiotics" refer to a mixture comprising microorganisms and substrate(s) selectively utilized by host microorganisms that confers a health benefit on the host, e.g., a combination of probiotics and prebiotic fibres.

### Prebiotics

The combination according to the invention comprises galacto-oligosaccharides (GOS) and fructo-oligosaccharides (FOS). GOS are preferably selected from the group consisting of betagalacto-oligosaccharides, alphagalacto-oligosaccharides, and galactan. According to a preferred embodiment GOS are betagalacto-oligosaccharides. Preferably the GOS comprise galacto-oligosaccharides with beta(1,4), beta(1,3) and/or beta(1,6) glycosidic bonds and a terminal glucose. Transgalacto-oligosaccharides is for example available under the trade name Vivinal^{®}GOS (Domo FrieslandCampina Ingredients), Bi2muno (Clasado), Cup-oligo (Nissin Sugar), Oligomate55 (Yakult), Promovita (Dairy Crest), Bioligo (Ingredion).

Fructo-oligosaccharides may in other context have names like fructopolysaccharides, oligofructose, polyfructose, polyfructan, inulin, levan and fructan and may refer to oligosaccharides comprising beta-linked fructose units, which are preferably linked-by beta(2,1)-and/or beta(2,6) glycosidic linkages, and a preferable DP between 2 and 200. Preferably, the fructo-oligosaccharides contain a terminal beta(2,1) glycosidic linked glucose. Preferably, the fructo-oligosaccharides contain at least 7 beta-linked fructose units. Inulin is a type of fructo-oligosaccharides wherein at least 75% of the glycosidic linkages are beta(2,1) linkages. Typically, inulin has an average chain length between 8 and 60 monosaccharide units. Preferred fructo-oligosaccharides for use in the combination of the present invention is commercially available under the trade names Raftiline^{®}HP (Orafti), Fibrulose and Fibruline (Cosucra) and Frutafit and Frutalose (Sensus).

The present combination comprises a mixture of GOS and FOS. Preferably the mixture of GOS and FOS is present in a weight ratio of from 1/99 to 99/1, more preferably from 1/19 to 19/1, more preferably from 1/1 to 19/1, more preferably from 2/1 to 15/1, more preferably from 5/1 to 12/1, even more preferably from 8/1 to 10/1, even more preferably in a ratio of about 9/1. This weight ratio is particularly advantageous when the GOS have a low average DP and FOS have a relatively high DP.

Preferably the GOS are short-chain galacto-oligosaccharides (scGOS) and the FOS are long-chain fructo-oligosaccharides (IcFOS). Most preferred is a mixture of GOS with an average DP below 10, preferably below 6, and FOS with an average DP above 7, preferably above 11, even more preferably above 20. Preferably the GOS have an average DP in the range from 3-7 and the FOS have an average DP in the range from 20-40. Preferably, GOS and FOS are present in a weight ratio in the range from 5:1 to 12: 1, preferably in a weight ratio of from 8 : 1 to 10 : 1.

### Lacticaseibacillus ssp.

The combination according to the invention comprises *Lacticaseibacillus ssp..* In a preferred embodiment, the *Lacticaseibacillus ssp.* is *Lacticaseibacillus* paracasei. Commercially available *Lacticaseibacillus paracasei* include e.g. *Lacticaseibacillus ssp.* LPC-37 (ATCC 334).

The subject according to the invention is an infant, a toddler, a child, a teenager, an adult or an elderly subject. Preferably, the subject is 12 months to 12 years old, 12-18 years old, 18-45 years old.

The combination for use according to the invention may be used on its own or may be comprised in a composition, such as a nutritional composition. The combination for use according to the present invention is preferably comprised in a nutritional composition. Hereafter the combination comprised in a nutritional composition is also referred to as the nutritional composition for use according to the invention or the nutritional composition according to the invention or the present nutritional composition.

In one embodiment, the nutritional composition is directed to infant or clinical nutrition. Preferably, the nutritional composition-is an infant formula, follow on formula, growing up milk, fortified milk, milk supplement, and the like. Preferably, the nutritional composition is an infant formula or follow on formula. The nutritional composition can be advantageously applied as a complete nutrition for infants. Preferably the present nutritional composition is an infant formula. An infant formula is defined as a formula for use in infants and can for example be a starter formula, intended for infants of 0 to 6 or 0 to 4 months of age. A follow on formula is intended for infants of 4 or 6 months to 12 months of age. At this age infants start weaning on other food. A toddler or growing up milk or formula is intended for children of 12 to 36 months of age. The present nutritional composition preferably comprises a lipid component, protein component and carbohydrate component and is preferably administered in liquid form. The present nutritional composition may also be in the form of a dry food, preferably in the form of a powder which is accompanied with instructions as to mix said dry food, preferably powder, with a suitable liquid, preferably water. The present nutritional composition preferably comprises other fractions, such as vitamins, minerals, trace elements and other micronutrients in order to make it a complete nutritional composition. Preferably infant formulas comprise vitamins, minerals, trace elements and other micronutrients according to international directives.

In some embodiments, the combination is suitable to be comprised in composition for children from 36 months to 18 years of age. The combination according to the invention can be included in powdered compositions to be dissolved in water, milk or juice, capsules or pills, saches, liquid drinks, fortified drinks, supplements, nutraceutical gums and jellies, weaning foods, semi-solid or solid foods, and the like.

In some embodiments the combination is comprised in a supplement or therapeutic compositions directed to adults, and optionally comprising further pharmaceutically or nutritionally accepted adjuvants.

The nutritional composition according to the invention preferably comprises 0.13 - 2.7 g GOS per 100 ml, more preferably 0.20 - 2.0 g, even more preferably 0.40 - 1.3 g GOS per 100 ml as a drink or as a powder in reconstituted ready-to-drink form. Based on dry weight, the nutritional composition according to the invention preferably comprises 1.0 - 20 g GOS per 100 g, more preferably 1.5 - 15 g, even more preferably 3.0 - 10 g GOS per 100 g. Based on calories, the nutritional composition according to the invention preferably comprises 0.2 - 4.0 g beta galactooligosaccharides per 100 kcal, more preferably 0.3 - 3.0 g, even more preferably 0.6 - 2.0 g GOS per 100 kcal. Preferably, GOS is beta-galactooligosaccharides.

Furthermore, the nutritional composition according to the invention preferably comprises 0.02- 1.5 g FOS per 100 ml, more preferably 0.04- 1.0 g, even more preferably 0. 08- 0.5 g FOS per 100 ml. Based on dry weight, the nutritional composition according to the invention preferably comprises 0.1 - 2.0 g FOS per_ 100 g, more preferably 0.2 -1.5 g, even more preferably 0.4- 1.0 g FOS per 100 g. Based on calories, the nutritional composition according to the invention preferably comprises 0.02 - 0.4 g FOS per 100 kcal, more preferably 0.04- 0.6 g, even more preferably 0.06 - 0.3 g FOS per 100 kcal.

The present nutritional composition comprises preferably 0.5 to 20 wt% of the combination of GOS, FOS, more preferably 1.5 to 15 wt%, even more preferably 2.5 to 12 wt%, most preferably 5.0 to 10.0 wt%, based on dry weight of the nutritional composition. Based on 100 ml the present nutritional composition preferably comprises 0.35 to 2.5 wt% combination of GOS, FOS, more preferably 0.35 to 2.0 wt%, even more preferably 0.4 to 1.5 wt%, based on 100 ml of the nutritional composition. A lower amount of the combination will be less effective in preventing or treating the conditions of the invention, whereas a too high amount will result in side-effects of bloating and abdominal discomfort.

Preferably, the present nutritional composition comprises 2 ×10³ to 3 × 10¹² colony forming units (cfu) *Lacticaseibacillus ssp.* per gram dry weight of the nutritional composition, more preferably 2 x 10⁴ to 3 × 10¹¹ colony forming units (cfu) *Lacticaseibacillus ssp.* per gram dry weight, even more preferably 2 × 10⁵ to 3 × 10⁹ colony forming units (cfu) *Lacticaseibacillus ssp.* per gram dry weight, most preferably 3 × 10⁵ to 3 × 10⁸ colony forming units (cfu) *Lacticaseibacillus ssp.* per gram dry weight of nutritional composition.

In one embodiment, the nutritional composition further comprises non digestible oligosaccharides other than GOS and FOS. Such other NDOs are preferably selected from the group consisting of human milk oligosaccharides (HMO), such as 2'-FL, 3'-SL, 6SL, and the like.

The present nutritional composition preferably comprises lipid, protein and digestible carbohydrate wherein the lipid provides 5 to 50% of the total calories, the protein provides 5 to 50% of the total calories, and the digestible carbohydrate provides 15 to 90% of the total calories. Preferably, in the present nutritional composition the lipid provides 35 to 50% of the total calories, the protein provides 7.0 to 12.5% of the total calories, and the digestible carbohydrate provides 40 to 55% of the total calories. For calculation of the % of total calories for the protein, the total of energy provided by proteins, peptides and amino acids needs to be taken into account. Preferably the lipid provides 3 to 7 g lipid per 100 kcal, preferably 4 to 6 g per 100 kcal, the protein provides 1.6 to 4 g per 100 kcal, preferably 1.7 to 2.5 g per 100 kcal and the digestible carbohydrate provides 5 to 20 g per 100 kcal, preferably 8 to 15 g per 100 kcal of the nutritional composition. Preferably the present nutritional composition comprises lipid providing 4 to 6 g per 100 kcal, protein providing 1.6 to 2.0 g per 100 kcal, more preferably 1.7 to 1.9 g per 100 kcal and digestible carbohydrate providing 8 to 15 g per 100 kcal of the nutritional composition. In one embodiment, the lipid provides 3 to 7 g lipid per 100 kcal, preferably 4 to 6 g per 100 kcal, the protein provides 1.6 to 2.1 g per 100 kcal, preferably 1.6 to 2.0 g per 100 kcal and the digestible carbohydrate provides 5 to 20 g per 100 kcal, preferably 8 to 15 g per 100 kcal of the nutritional composition and wherein preferably the digestible carbohydrate component comprises at least 60 wt.% lactose based on total digestible carbohydrate, more preferably at least 75 wt.%, even more preferably at least 90 wt.% lactose based on total digestible carbohydrate. The amount of total calories is determined by the sum of calories derived from protein, lipids, digestible carbohydrates and non-digestible oligosaccharide.

The present nutritional composition preferably comprises a digestible carbohydrate component. Preferred digestible carbohydrate components are lactose, glucose, sucrose, fructose, galactose, maltose, starch and maltodextrin. Lactose is the main digestible carbohydrate present in human milk. The present nutritional composition preferably comprises lactose. Preferably the present nutritional composition does not comprise high amounts of carbohydrates other than lactose. Compared to digestible carbohydrates such as maltodextrin, sucrose, glucose, maltose and other digestible carbohydrates with a high glycaemic index, lactose has a lower glycaemic index and is therefore preferred. The present nutritional composition preferably comprises digestible carbohydrate, wherein at least 35 wt.%, more preferably at least 50 wt.%, more preferably at least 60 wt.%, more preferably at least 75 wt.%, even more preferably at least 90 wt.% , most preferably at least 95 wt.% of the digestible carbohydrate is lactose. Based on dry weight the present nutritional composition preferably comprises at least 25 wt.% lactose, preferably at least 40 wt.%, more preferably at least 50 wt.% lactose.

The present nutritional composition preferably comprises at least one lipid selected from the group consisting of animal lipid (excluding human lipids) and vegetable lipids. Preferably the present nutritional composition comprises a combination of vegetable lipids and at least one oil selected from the group consisting of fish oil, animal oil (including animal fat, for instance milk fat), algae oil, fungal oil, and bacterial oil. Herein, the terms oil and fat are interchangeably used. The lipid of the present nutritional composition preferably provides 3 to 7 g per 100 kcal of the nutritional composition, preferably the lipid provides 4 to 6 g per 100 kcal. When in liquid form, e.g. as a ready-to-feed liquid, the nutritional composition preferably comprises 2.1 to 6.5 g lipid per 100 ml, more preferably 3.0 to 4.0 g per 100 ml. Based on dry weight the present nutritional composition preferably comprises 12.5 to 40 wt.% lipid, more preferably 19 to 30 wt.%. Preferably the lipid comprises the essential fatty acids alpha-linolenic acid (ALA), linoleic acid (LA) and/or long chain polyunsaturated fatty acids (LC-PUFA). The LC-PUFA, LA and/or ALA may be provided as free fatty acids, in triglyceride form, in diglyceride form, in monoglyceride form, in phospholipid form, or as a mixture of one of more of the above. Preferably the present nutritional composition comprises at least one, preferably at least two lipid sources selected from the group consisting of rape seed oil (such as colza oil, low erucic acid rape seed oil and canola oil), high oleic sunflower oil, high oleic safflower oil, olive oil, marine oils, microbial oils, coconut oil, palm kernel oil. The present nutritional composition is not human milk.

The present nutritional composition preferably comprises protein. The protein used in the nutritional composition is preferably selected from the group consisting of-non-human animal proteins, preferably milk proteins, vegetable proteins, such as preferably soy protein and/or rice protein, and mixtures thereof. The present nutritional composition preferably contains casein, and/or whey protein, more preferably bovine whey proteins and/or bovine casein. Thus, in one embodiment the protein in the present nutritional composition comprises protein selected from the group consisting of whey protein and casein, preferably whey protein and casein, preferably the whey protein and/or casein is from cow's milk. Preferably the protein comprises less than 5 wt.% based on total protein of free amino acids, dipeptides, tripeptides or hydrolyzed protein. The present nutritional composition preferably comprises casein and whey proteins in a weight ratio casein: whey protein of 10 : 90 to 90 : 10, more preferably 20 : 80 to 80 : 20, even more preferably 35 : 65 to 55 : 45.

The wt.% protein based on dry weight of the present nutritional composition is calculated according to the Kjeldahl-method by measuring total nitrogen and using a conversion factor of 6.38 in case of casein, or a conversion factor of 6.25 for other proteins than casein. The term 'protein' or 'protein component' as used in the present invention refers to the sum of proteins, peptides and free amino acids.

The nutritional composition preferably comprises 0.8 - 2.7 g protein per 100 ml, more preferably 0.9 - 2.1 g per 100 ml, more preferably 1.1- 1.6 g per 100 ml nutritional composition. Based on dry weight, the nutritional composition preferably comprises 6 - 20 g protein per 100 g, more preferably 7 - 16 g per 100 g, more preferably 8 - 12 g protein per 100 g dry weight of the nutritional composition. Based on calories, the nutritional composition preferably comprises 1.2 - 4.0 g protein per 100 kcal, more preferably 1.4- 3.2 g per 100 kcal, more preferably 1.6- 2.4 g protein per 100 kcal of the nutritional composition. The protein preferably provides 5 - 15%, more preferably 6 - 13% even more preferably 7 - 10% based on total calories of the composition. Protein is to be taken as the sum of proteins, peptides, and free amino acids. The amount of protein can be calculated according to the amount of nitrogen multiplied by 6.25. The present nutritional composition preferably comprises casein and/or whey proteins. Preferably the weight ratio casein:whey protein is 0:100 to 90:10, more preferably 20:80 to 90:10, more preferably 40:60 to 80:20.

In order to meet the caloric requirements of an infant or toddler, when the composition is of the infant formulae type, the nutritional composition preferably comprises 45 to 200 kcal/100 ml liquid. For infants the nutritional composition has more preferably 60 to 90 kcal/100 ml liquid, even more preferably 65 to 75 kcal/100 ml liquid. This caloric density ensures an optimal ratio between water and calorie consumption. For toddlers, human subjects with an age between 12 and 36 months, the nutritional composition more preferably has a caloric density between 45 and 65, even more preferably between 50 and 60 kcal/100 ml. The osmolarity of the present composition is preferably between 150 and 420 mOsmol/l, more preferably 260 to 320 mOsmol/l. The low osmolarity aims to further reduce the gastro-intestinal stress.

When the nutritional composition is in a ready to feed, liquid form, the preferred volume administered on a daily basis is in the range of about 80 to 2500 ml, more preferably about 200 to 1200 ml per day. Preferably, the number of feedings per day is between 1 and 10, preferably between 3 and 8. In one embodiment the nutritional composition is administered daily for a period of at least 2 days, preferably for a period of at least 4 weeks, preferably for a period of at least 8 weeks, more preferably for a period of at 25 least 12 weeks, in a liquid form wherein the total volume administered daily is between 200 ml and 1200 ml and wherein the number of feedings per day is between 1 and 10.

In a second aspect, the present invention concerns a composition comprising, based on dry weight, 6 - 20 g protein per 100g of the composition, 2 × 10³ to 3 × 10¹² colony forming units (cfu) *Lacticaseibacillus ssp.* per gram of the composition, and 1-20 g of a combination of GOS and FOS per 100g of the composition.

Preferably, the composition further comprises a protein selected from casein, whey or a plant-based protein.

In a preferred embodiment, the composition comprises, on dry weight, 0.1 - 2.0 g FOS per 100 g of the composition and/or 1.0 - 20 g GOS per 100 g of the composition.

The combination(s) and composition(s) according to the invention are preferably enterally administered, more preferably orally.

### EXAMPLES

### Example 1

To test the effect of the combination according to the invention in dysbiosis induced by proteolytic fermentation, faecal samples from healthy children (18-24 months old) were used in an *in vitro* fermentation model to induce proteolysis thereby mimicking the dysbiotic pattern found in the gut microbiota of patients with protein digestion problems. Proteolysis was induced by adding tryptone (20 g/L concentration) for 24 hours.

A combination according to the invention ("A") comprising 0.5% w/w scGOS/lcFOS (ratio 9:1) and 10⁷-10⁸ cfu/ml of *Lacticaseibacillus paracasei* was tested and compared with a reference composition ("Ref") comprising 0.5% w/w Synergy1 (commercially available prebiotic fiber comprising oligofructose-enriched inulin) and 10⁷-10⁸ cfu/ml *L. paracasei.* -The scGOS was VivinalGOS^{®} the IcFOS was RaftilineHP^{®}.

Test started after 24 tryptone fermentation (t0) by adding the respective test compositions. The total test time was 56 hours. To reach a steady state, mixtures were added every 4 hours during the day and once in the end of the day for overnight. The 4h intervals allow all fibers to be utilized by the microbial community and reach a steady state (data not shown). Samples were collected in the beginning (t0) and at the end of test (t56h) for microbiota profiling analysis (16S rRNA sequencing).

Under anaerobic conditions, the faecal samples were thawed and approximately a 10% (w/v) suspension of the fecal samples were made in age adapted Colonic Microbiota medium containing 25 mM acetate and 12 mM lactate, 25 mg/L bile acids (Sigma), 15 mmol/L ammonium sulphate without carbon source adjusted to toddler stool pH (6.5) in order to mimic uncompromised intestinal conditions. The diluted faecal samples were homogenized, allowed to sediment for 5 minutes, and subsequently filtered over a Millex 100 µm vacuum filter. The filtered fecal samples were pooled together in 1:1:1 ratio. A Biolector Pro plate (BOH2 round well, M2P-labs) with pH optodes was used. One feeding row of the plate was filled with sterile 3M NaOH for the pH adjustment. Thirty-two wells of the plate were filled with 800 µL of the pooled faecal solution (10% w/v) and 800 µL of Tryptone (40 g/L) were added to the wells with the faecal samples. After this, the plates were sealed with ventilated silicone foil with slits. The plate was incubated (85% moisture, 37 degrees, 600 rpm, anaerobic) in BioLector Pro for 24 hours. At the end of the protein fermentation, the experiment was paused, the faecal slurry of each well was harvested and shortly centrifuged, under α-septical anaerobic conditions. The supernatant was frozen for further analyses, three faecal pellets were frozen for 16S rRNA sequencing analysis while the rest of the faecal pellets was resuspended into 800 µL of fresh colonic microbiota medium and pipetted back in the wells of a new BOH2 plate.

At the beginning of the fermentation, 800 µL of sterile water solution was added to control wells, while sterile GOS/FOS (1%) and Synergy1 tested solutions were added to the respective test wells. To the wells with GOS/FOS and Synergy1, the cells of active culture of *L. paracasei* were added in a concentration of 2x10E7 CFUs/mL. The experiment was started with pH at setpoint 6.5 for toddler samples with continuous pH control. After 4 hours the experiment was paused, the faecal slurry of each well was harvested and shortly centrifuged, the supernatant was frozen for further analyses while the faecal pellet was resuspended in fresh medium with carbohydrates and *L. paracasei* cells and pipetted back in the original well of BOH2 plate. The procedure was repeated for 3 days, with 3 times manual feeding every 4 hours per day. Subsequently, the faecal pellets from the last time point (t56) were stored for 16S rRNA sequencing analysis.

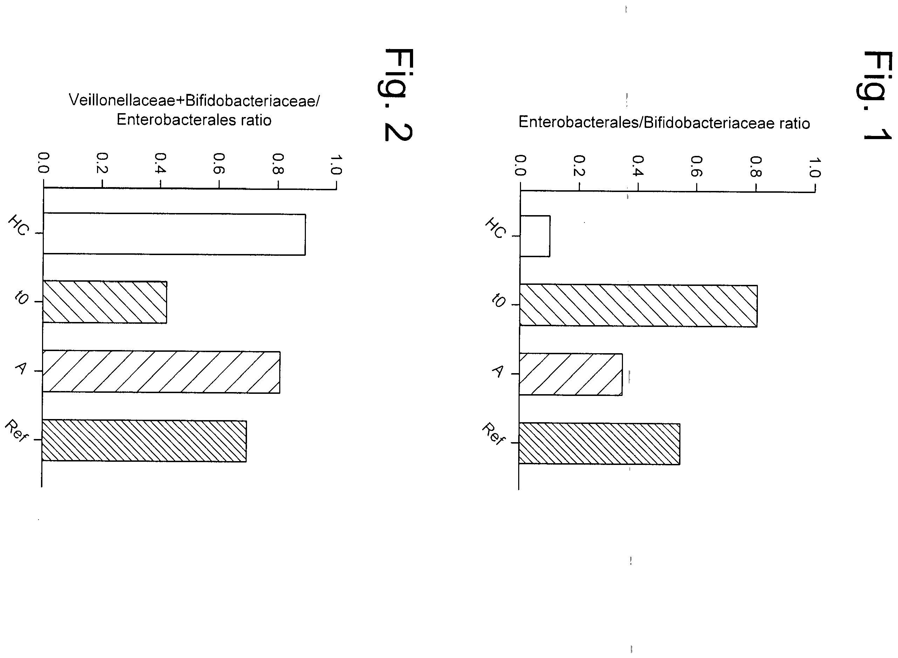

The results of the combination according to the invention *versus* the comparative combination is shown in Figure 1. The results show that the composition according to the invention is advantageously capable of modulating the gut microbiota in dysbiotic state induced by proteolytic fermentation. In particularly, in Figure 1, it is shown that a synbiotic composition according to the invention substantially reduced the ratio of Enterobacterales to Bifidobacteriaceae when compared to the synbiotic composition comprising reference composition (Synergy1 and *L. paracasei*)*,* thus bringing the ratio closer to that of healthy controls (HC), ie, prior to the disturbance caused by the proteolytic fermentation.

It was further observed that the ratio of Veillonellaceae + Bifidobacteriaceae over Enterobacterales is closer to the healthy controls after synbiotics treatment, when compared to the reference combination (Fig. 2).

### Example 3

A powdered nutritional composition for young children, which after reconstitution with water (1 scoop of 14.8g powder) provides a ready to feed liquid composition comprising per 100 ml:
- 67 kcal
- 1.3 g protein (milk protein)
- 3.4 g lipids (mainly vegetable lipids)
- 7.3 g digestible carbohydrate (mainly lactose)
- 1.15 g of scGOS (source VivinalGOS) and IcFOS (source RaftilinHP) in a 9:1 weight ratio.
- about 10⁹ cfu *Lacticaseibacillus paracasei* strain 37
- Vitamins and minerals as known in the art.

### Example 4

Supplement to be added to milk, yoghurt, porridge and the like. Sachet comprising per pack:
- 4 g fibres,
- 2.1 g Galacto-oligosaccharides (VivinalGOS powder),
- 0.5 g FOS (Raftiline HP),
- about 10⁹ cfu of *L. paracasei* LPC37.

## Claims

1. A combination of galacto-oligosaccharides and fructo-oligosaccharides with *Lacticaseibacillus ssp.* for use in preventing and/or treating impaired gut health associated with a gut-brain-axis condition in a subject in need thereof.

2. The combination for use according to claim 1, wherein impaired gut health is selected from gut dysbiosis, gut dysfunction, fragile gut, and/or impaired protein digestion.

3. The combination for use according to claim 1 or 2, wherein the gut brain axis condition is selected from autism spectrum disorders (ASDs), wherein ASDs are preferably selected from autism disorder (AD), Asperger's syndrome and pervasive developmental disorder-not otherwise specified (PDD-NOS).

4. The combination for use according to any of the preceding claims, wherein the subject is a neurodivergent subject or has a neurodivergent sibling.

5. The combination for use according to any of the preceding claims, wherein the subject suffers from at least one atypical eating behaviour.

6. The combination for use according to any of the preceding claims, wherein preventing and/or treating impaired gut health associated with a gut-brain-axis condition comprises improving the gut microbiota of the subject.

7. The combination for use according to claim 6, wherein improving gut microbiota comprises at least one of:
a. increasing the relative abundance of saccharolytic bacterial taxa, preferably selected from Bifidobacteriaceae, Lactobacillaceae, Lachnospiraceae and Veillonellaceae;
b. decreasing the relative abundance of proteolytic bacterial taxa, preferably selected from Enterobacterales, Peptostreptococcaceae, Peptostreptococcales-tissirierallales, Oscillospiraceae and Eggerthellaceae;
c. decreasing the ratio of Enterobacterales to Bifidobacteriaceae; or
d. increasing the ratio of Veillonellaceae plus Bifidobacteriaceae to Enterobacterales.

8. The combination for use according to any of the preceding claims, wherein improving gut microbiota comprises decreasing the ratio of Enterobacterales to Bifidobacteriaceae and/or increasing the ratio of Veillonellaceae plus Bifidobacteriaceae to Enterobacterales.

9. The combination for use according to any of the preceding claims, wherein improving gut microbiota comprises decreasing the ratio of Enterobacterales to Bifidobacteriaceae.

10. The combination for use according to any of the preceding claims, wherein *Lacticaseibacillus ssp.* is *Lacticaseibacillus paracasei.*

11. The combination for use according to any of the preceding claims, wherein the galacto-oligosaccharides have an average degree of polymerisation in the range from 3-7 and the fructo-oligosaccharides have an average degree of polymerisation in the range from 20-40.

12. The combination for use according to any of the preceding claims, wherein the weight ratio of galacto-oligosaccharides to fructo-oligosaccharides is in the range from 5 : 1 to 12 : 1, preferably in the range from 8 : 1 to 10 : 1.

13. The combination for use according to any of the preceding claims, wherein the combination is comprised in a nutritional composition, preferably in an infant formula or follow on formula.

14. The combination for use according to any of the preceding claims, wherein the combination is comprised in a nutritional composition in an amount of 0.5 to 20 wt%, preferably 1.5 to 15 wt%, more preferably 2.5 to 12 wt%, even more preferably 5.0 to 10.0 wt%, based on dry weight of the nutritional composition.

15. A composition comprising, based on dry weight, 6 - 20 g protein per 100g of the composition, 2 × 10³ to 3 × 10¹² colony forming units (cfu) *Lacticaseibacillus ssp.* per gram of the composition, and 1-20 g of a combination of GOS and FOS per 100g of the composition, preferably wherein the composition comprises, on dry weight, 0.1 - 2.0 g FOS per 100 g of the composition and/or 1.0 - 20 g GOS per 100 g of the composition.
